# EUROPEAN PATENT SPECIFICATION

(11) **EP 1 341 532 B1**
(45) Date of publication and mention of the grant of the patent: **26.07.2006**
(21) Application number: 01992569.2
(22) Date of filing: 02.11.2001
(51) Int. Cl.: A61K 31/00, A61P 3/06

(54) **USE OF PYRIDOXAMINE FOR THE TREATMENT AND INHIBITION OF OBESITY-RELATED COMPLICATIONS**
VERWENDUNG VON PYRIDOXAMIN FÜR DIE BEHANDLUNG UND UNTERDRÜCKUNG VON KOMPLIKATIONEN IM ZUSAMMENHANG MIT ADIPOSITAS
UTILISATION DE PYRIDOXAMINE POUR TRAITER ET EMPECHER LE DEVELOPPEMENT DE COMPLICATIONS ASSOCIEES A L'OBESITE

(30) Priority: 02.11.2000 US 245630 P; 29.08.2001 US 315789 P
(43) Date of publication of application: 10.09.2003
(73) Proprietor: UNIVERSITY OF SOUTH CAROLINA, Columbia, SC 29208 (US)
(72) Inventor: BAYNES, John, Columbia, SC 29205 (US)
(74) Representative: Vossius, Volker
(86) International application number: PCT/US2001/045415
(87) International publication number: WO 2002/036109

(56) References cited:
- WO-A-00/21516
- WO-A-00/23063
- WO-A-99/25690
- ALDERSON NATHAN A ET AL: "Pyridoxamine (PM) inhibits dyslipidemia, hypertension, formation of advanced glycoxidation and lipoxidation end-products (AGEs/ALEs), and development of renal and vascular pathology in the Zucker obese (fa/fa) rat." DIABETES, vol. 50, no. Supplement 2, June 2001 (2001-06), page A172 XP001098560 61st Scientific Sessions of the American Diabetes Association;Philadelphia, Pennsylvania, USA; June 22-26, 2001 ISSN: 0012-1797
- KAWADA, TERUO; AOKI, NAOHITO; KAMEI, YASUTOMI; MAESHIGE, KATSUHIKO;NISHIU, SACHIKO; SUGIMOTO, ETSURO: "Comparative investigation of vitamins and their analogs on terminal differentiation, from preadipocytes to adipocytes, of 3T3-L1 cells" COMP. BIOCHEM. PHYSIOL, vol. 96A, no. 2, 1990, pages 323-326, XP001085187
- NANBARA S ET AL: "CHANGES ON LEVELS OF B-6 VITAMIN AND AMINOTRANSFERASE IN THE LIVER OF DIABETIC ANIMALS" DIABETES RESEARCH AND CLINICAL PRACTICE, vol. 9, no. 2, 1990, pages 109-114, XP001084802 ISSN: 0168-8227

## Description

### Cross-Reference

This application claims priority form U.S. Provisional Patent Application Serial Nos. 60/245,630, filed November 2, 2000, and 60/315,789 filed August 29, 2001.

### Statement of Government Support

This work was supported by a research grant from the National Institute of Diabetes, Digestive, and Kidney Disease (DK-19971).

### Field of the Invention

The invention is related to the fields of obesity and obesity complications.

### Background of the Invention

Obesity is generally defined as a body mass index (BMI) of greater than 30 kg/m² (m² = the square of height measured in meters) while morbid or extreme obesity is generally defined as a BMI of greater than 40 kg/m². Both types of obesity represent a health hazard, and are associated with a host of other disorders, including but not limited to hypertension, dyslipidemia, and insulin resistance and/or hyperinsulinemia. Normal treatment for morbid obesity is surgery to remove part of the stomach or intestine.

This combination of disorders is also seen in many cases of metabolic syndrome X, which is a multifactorial syndrome that occurs frequently in the general population. Such manifestations of metabolic syndrome X have been shown to increase the risk of coronary artery disease. Obesity as a causal factor of metabolic X syndrome is bolstered by the extraordinary efficacy of obesity prevention to prevent the entire syndrome. Thus, there is a need in the art for methods of treating obese subjects to minimize these obesity-related complications.

Pyridoxamine has previously been shown to inhibit the formation of advanced glycation end-products both in vitro and in diabetic animal models, and to be effective in treating and preventing diabetic nephropathy (U.S. Patent No. 5,985,857; WO 00/21516) Pyridoxamine has also been shown to be effective in treating and preventing hyperlipidemia in diabetic animal models (WO 00/23063). However, it is not known whether pyridoxamine is effective for treating obese patients for the purpose of treating or inhibiting obesity-related complications, such as those described above.

### Summary of the Invention

The present invention is directed to the use of pyridoxamine for the preparation of a pharmaceutical composition for treating or inhibiting obesity-related complications in an obese subject, wherein the obesity-related complications are selected from the group consisting of hyperlipidemia, renal disease, hypertension, and advanced lipoxidation end-product formation. In one embodiment the composition is to inhibit such obesity-related complications; in another embodiment the composition is to treat such obesity-related complications. In various further embodiments of the methods, the obese subject (individually or in combination): is morbidly obese; is not hyperglycemic; is insulin-resistant; and suffers from a leptin receptor defect.

### Detailed Description of the Invention

Advanced glycation end-products (AGEs) are carbohydrate-derived chemical modifications and crosslinks that accumulate in long-lived tissue proteins during normal aging (Thorpe and Baynes, Drugs & Aging 9:69-77 (1996)). The increased rate of accumulation of AGEs during hyperglycemia is implicated in the development of long-term complications of diabetes (Baynes and Thorpe, In: Diabetes in the New Millenium, Turtle et al. Eds., Endocrinology and Diabetes Research Foundation, Sydney, pp. 337-350 (1999); Ulrich et al., Recent Prog. Horm. Res. 56:1-21 (2001)). We have recently demonstrated that the AGE inhibitor pyridoxamine (Khalifah et al., Biochem. Biophys. Res. Commun. 257:251-258 (1999)) significantly inhibited the development of renal disease in the streptozotocin (STZ)-induced diabetic rat. Pyridoxamine had no effect on glycemic control, but caused a marked reduction in albuminuria and plasma creatinine, accompanied by decreased crosslinking and levels of the AGEs N^{ε}-(carboxymethyl)lysine (CML) and N^{ε-}(carboxyethyl)lysine (CEL), but not pentosidine, in skin collagen. However, in addition to these changes, there was an unanticipated, >50% reduction in hyperlipidemia in the STZ-rat, which correlated significantly with the improvement in renal function. Thus, it was not clear whether the inhibition of renal disease by pyridoxamine was secondary to the correction of hyperlipidemia or the decrease in AGEs, or if the decrease in AGEs and hyperlipidemia were secondary to effects of pyridoxamine on renal function. In other studies we had observed that pyridoxamine was not only an AGE inhibitor, but also a potent inhibitor of formation of advanced lipoxidation end-products (ALEs) during lipid peroxidation reactions *in vitro* (Onorato et al., J. Biol. Chem. 275:21177-21184 (2000)). Since hyperglycemia was unaffected by pyridoxamine, and CML and CEL, but not pentosidine, may be formed from lipids, as well as from carbohydrates (Fu et al., J. Biol. Chem. 271:9982-9986 (1996)), we considered the possibility that CML and CEL, as well as crosslinking of collagen, in the STZ-diabetic rat might result from advanced lipoxidation reactions and that pyridoxamine might be working as both a hypolipidemic agent and an inhibitor of ALE formation in this model.

The present study was undertaken to evaluate whether pyridoxamine would be useful for limiting or preventing renal disease in an obese, hyperlipidemic, and non-diabetic animal model of nephropathy. The Zucker fa/fa rat was chosen for these studies because of its obesity and chronic hyperlipidemia, resulting from a homozygous defect in the leptin receptor, the absence of hyperglycemia, and its susceptibility to development of chronic renal disease (Kasiske et al., Hypertension 19(1 Suppl):Il 10-I115 (1992)). The Zucker fa/fa rat has a defect in the leptin receptor, an event that leads to extreme obesity in humans (Leibel et al., J. Biol. Chem., 272(51):31937-40). The Zucker rat is a model for the pre-diabetic state, characterized by hypertension, insulin resistance, and hyperlipidemia, and thus also model for metabolic syndrome X.

In this study, we show that pyridoxamine significantly inhibits the development of hyperlipidemia, the chemical modification of proteins by AGE/ALEs, the development of renal and vascular pathology, and hypertension in this animal model.

As used herein "obesity" is generally defined as a body mass index (BMI) of greater than 30 kg/m².

As used herein, "hyperlipidemia" includes both hypertriglyceridemia and hypercholesterolemia. In a preferred embodiment, hypertriglyceridemia refers to fasting triglyceride level of greater than 150 mg/dl in human subjects; more preferably greater than 200 mg/dl.

As used herein, "hypertension" refers to an increased blood pressure over that normally seen in a subject of a particular age and set of other risk factors. In a non-limiting example, hypertension is defined by a systolic blood pressure of greater than or equal to 140 mmHg, and a diastolic blood pressure of 90 mmHg or greater.

As used herein, the term "renal disease" refers to one or more of proteinuria (increased urinary protein, as measured, for example, by albuminuria), a decrease in renal function (i.e.: impaired glomerular clearance; as measured, for example, by creatinine clearance), and nephropathy (defined as the combination of proteinuria and albuminuria together with hypertension).

In one embodiment, the obese subject is morbidly obese. As used herein, the term "morbidly obese" or "morbid obesity" is defined as a BMI of greater than 40 kg/m².

In another embodiment, the obese subject is not hyperglycemic.

In another embodiment, the obese subject is insulin-resistant. As used herein, insulin resistance refers to a state in which normal concentrations of insulin produces a less than normal biological response to insulin.

The obese subject may have metabolic syndrome X. As used herein, "metabolic syndrome X" refers to a combination of obesity or morbid obesity and insulin resistance, which can also be accompanied by hypertension and/or hyperlipidemia.

In another embodiment, the obese subject suffers from a leptin receptor defect. As used herein, a "leptin receptor defect" refers to either an inability to properly transmit leptin signaling, including leptin receptor defects caused by genetic defects in the leptin receptor that result in increased, diminished, or no production of leptin receptor; or production of mutated leptin receptor that either does not function properly or does not function optimally.

Alternatively, the obese subject suffers from a "leptin defect", including defects resulting from increased, diminished, or no production of leptin, or production of mutated leptin that either does not function properly or does not function optimally.

Pyridoxamine can be administered as the sole active pharmaceutical agent, or it can be used in combination with one or more other agents useful for treating or preventing obesity-related complications. When administered as a combination, the therapeutic agents can be formulated as separate compositions that are given at the same time or different times, or the therapeutic agents can be given as a single composition.

The pyridoxamine may be made up in a solid form (including granules, powders or suppositories) or in a liquid form (*e*.*g*., solutions, suspensions, or emulsions). Pyridoxamine may be applied in a variety of solutions and may be subjected to conventional pharmaceutical operations such as sterilization and/or may contain conventional adjuvants, such as preservatives, stabilizers, wetting agents, emulsifiers, buffers etc.

For administration, the pyridoxamine is ordinarily combined with one or more adjuvants appropriate for the indicated route of administration. Pyridoxamine may be admixed with lactose, sucrose, starch powder, cellulose esters of alkanoic acids, stearic acid, talc, magnesium stearate, magnesium oxide, sodium and calcium salts of phosphoric and sulphuric acids, acacia, gelatin, sodium alginate, polyvinylpyrrolidine, and/or polyvinyl alcohol, and tableted or encapsulated for conventional administration. Alternatively, the pyridoxamine may be dissolved in saline, water, polyethylene glycol, propylene glycol, carboxymethyl cellulose colloidal solutions, ethanol, corn oil, peanut oil, cottonseed oil, sesame oil, tragacanth gum, and/or various buffers. Other adjuvants and modes of administration are well known in the pharmaceutical art. The carrier or diluent may include time delay material, such as glyceryl monostearate or glyceryl distearate alone or with a wax, or other materials well known in the art.

Pharmaceutical compositions containing pyridoxamine are administered to an obese individual in need thereof. In therapeutic applications, pyridoxamine is administered to an obese mammalian patient in an amount sufficient to reduce or inhibit obesity-related complications selected from the group consisting of hyperlipidemia, renal disease, hypertension and advanced lipoxidation end-product formation. Amounts effective for this use depend on factors including, but not limited to, the route of administration, the stage and severity of the obesity and/or obesity-related complications, the general state of health of the mammal, and the judgment of the prescribing physician. Pyridoxamine is safe and effective over a wide dosage range. However, it will be understood that the amount of pyridoxamine actually administered will be determined by a physician, in the light of the above relevant circumstances.

Pyridoxamine may be administered by any suitable route, including orally, parentally, by inhalation or rectally in dosage unit formulations containing conventional pharmaceutically acceptable carriers, adjuvants, and vehicles, including liposomes. The term parenteral as used herein includes subcutaneous, intravenous, intraarterial, intramuscular, intrasternal, intratendinous, intraspinal, intracranial, intrathoracic, infusion techniques, intracavity, or intraperitoneally. Preferably, pyridoxamine is administered orally or parentally.

### Examples

### MATERIALS AND METHODS

Materials. Pyridoxamine (PM)(HCl)₂ was provided by Biostratum, Inc. (Research Triangle Park, NC). Assay kits for plasma metabolites were from Sigma (St. Louis, MO), unless otherwise noted.

Animals and treatments: All experiments were carried out in accordance with the regulations of the Institutional Animal Care and Use Committee of the University of South Carolina. Female Zucker obese (fa/fa) rats 6 weeks old, (n = 14) and lean (LN) littermates (Fa/fa), also 6 weeks old (n = 7) were purchased from Charles River Laboratories (Wilmington, MA). The fa/fa rats were divided randomly into two groups of 7 each, untreated fatty control (FC) and PM-treated (FPM) animals, the latter group receiving 2 g PM/L in the drinking water. The dose of PM was chosen to be consistent with the dose used in a previous study in non-diabetic Sprague-Dawley rats, and was well tolerated by both the Sprague-Dawley and Zucker rats. All animals were housed individually with a light dark cycle of 12 hours each, and had free access to food and water.

Plasma and blood analyses. Blood was obtained from the tail vein, using heparinized microhematocrit tubes, and plasma recovered by centrifugation at 4°C. All assays for proteins and metabolites in plasma and urine were performed by microplate adaptations of current methods, using a Wallac Victor Model 1420 multilabel plate reader (Wallac, Inc., Gaithersburg, MD). Control experiments showed that PM, at concentrations present in plasma or urine, did not cause interference in any of the assays, except for urinary protein (see below). Plasma glucose was measured using the Trinder assay Kit (Sigma # 315) and total glycated hemoglobin (GlcHb) by boronate affinity chromatography (Sigma kit # 442-B). Total plasma triglycerides and cholesterol were analyzed by enzymatic, colorimetric, end-point assays using Sigma kits for triglycerides (#37, GPO Trinder) and total cholesterol (#352). Insulin was measured using a direct sandwich ELISA for rat insulin (ALPCO, Windham, NH). Plasma PM concentration was measured by reverse phase high performance liquid chromatography (RP-HPLC) with fluorescence detection, using 293/393 nm excitation and emission wavelengths.

Nephropathy. Progression of renal disease was assessed by measurements of plasma creatinine and urinary albumin and total urinary protein. Plasma creatinine concentration was measured by the Jaffé picric acid procedure, using Sigma kit # 555-A. For urine collections, rats were housed in metabolic rat cages for 24 hr with free access to food and water. Several drops of toluene were added to the urine collection beaker to inhibit microbial growth. Urinary albumin was quantified by an ELISA assay. Rabbit antiserum to rat albumin and horseradish peroxidase (HRP)-conjugated goat anti-rabbit IgG were purchased from ICN Biomedical Research Products, Costa Mesa, CA. Briefly, wells were coated with 50 ng of rat albumin (Sigma, St. Louis) in 0.1 M sodium carbonate buffer, pH 10.4, overnight at 4°C. In the competition step 100 µL of standard or diluted urine sample was incubated with 100 µL of rabbit anti-rat albumin antiserum for 3 hr at room temperature on a microplate shaker. After washing, horseradish peroxidase-conjugated goat anti-rabbit IgG (200 µL) was applied for 1 h at 25°C with shaking, and the plate was developed with 200 µL of ABTS-reagent (2,2'-azino-bis(3-ethylbenzthiazoline-6-sulfonic acid) for 45 min at room temperature. Absorbance was measured at 405 nm. Total urinary protein was measured using the Sigma Microprotein-PR Kit. Based on preliminary experiments in which known amounts of PM were added to urine, PM increased the final absorbance readings in the protein assay, so that a correction (≤ 10%) was applied for the PM content of the urine.

Blood pressure. Blood pressure measurements were initiated at week 5 in the experiment after acclimating the animals to the apparatus, and were carried out the week following blood and urine collection. Measurements were made by tail cuff plethysmography using a Kent Scientific XBP instrument.

Necropsy and tissue sampling. Blood was drawn from anaesthetized (isoflurane) rats by heart puncture and transferred into heparinized vacutainer tubes on ice. Plasma was separated from whole blood within 30 min by centrifugation in a refrigerated benchtop centrifuge; aliquots of plasma were stored at -70°C until time of analysis. Rats were then killed by over-anesthetization, and liver and kidneys were removed, and both organs rinsed in phosphate buffered saline, and weighed. One kidney was cut in half transversely, and one half was removed for fixation (buffered formalin) for light microscopy. The remaining kidney and liver samples were frozen at -70°C. The aorta was dissected out and fixed in buffered formalin, and a section of abdominal skin (2 cm²) was also removed, rinsed in phosphate buffered saline and stored at -70°C.

Assays of chemical modifications and fluorescence in skin collagen. The insoluble fraction of skin collagen was prepared by scraping the skin with a single-edged razor blade to remove hair and adventitious tissue, followed by a series of extractions with 0.5 M NaCl, 0.5 M acetic acid and chloroform/methanol (2:1 v/v), as described previously (Dyer et al., J. Clin. Invest. 91:2463-2469 (1993)). Fructoselysine (FL), a measure of glycation of collagen, and CML, CEL, MDA-lysine and HNE-lysine were assayed by isotope dilution, selected ion monitoring gas chromatography - mass spectrometry (SIM-GC/MS), and pentosidine by RP-HPLC, all as described previously (Requena et al., Biochem. J. 322:317-325 (1997)). Levels of AGEs/ALEs were expressed relative to the FL content of the collagen in order to correct for differences in mean age of the protein (see Table 1 and Discussion, below). Collagen fluorescence (Ex = 370nm, Em = 440 nm) was measured after solubilization with pepsin (20 ug/mL in 0.5 M acetic, 24 hr at 37°C), and was normalized to the hydroxyproline content of the collagen, measured as described by Stegemann and Stalder (Clin. Chim. Acta 18:267-273 (1967)).

Statistical analysis. Statistical analyses were performed using SigmaStat for Windows V1.00 (SPSS, Inc., Chicago, IL). P-values were calculated by non-parametric Mann-Whitney Rank Sum analysis. Correlation analyses were performed by the Pearson Product Moment method.

### RESULTS

Weight gain and glycemic control. Zucker obese rats already weighed significantly more than lean littermates at week 0 of the study (6 weeks of age; LN = 158.5 ± 10.8 g and fa/fa = 237 ± 14.3 g, respectively). At week 32, both groups of fa/fa rats weighed three times as much as LN rats (LN = 267 ± 26 g, FC = 721 ± 8 1 g, and FPM = 746 ± 60 g). There was no statistically significant difference in body weight between FC and FPM rats at any point in the study. PM also had no effect on glycemia. Despite the large differences in body weights between the LN vs. FC and FPM groups, mean fasting blood glucose was - 6.5 mM and mean total glycated hemoglobin ~ 6.8 % in all three groups throughout the 32 week experiment (Table 1). However, there were significant differences in collagen glycation between the LN and fatty groups, with FL levels increasing ~ 25% in the LN group with age, but declining about 35% in the fatty rats. FL increases modestly in human skin collagen with age, and is thought to reflect an age-dependent decrease in glucose tolerance. At the same time, the fatty rats are growing at a substantially greater rate than the LN animals, so that their total skin surface area and collagen mass is growing. Thus, on average, the skin collagen in the FC and FPM rats is younger, compared to that of the LN animals, and, although blood sugar was the same among the groups, the slow rate of glycation of collagen, combined with the lower mean age of skin collagen in the FC and FPM rats results in lower FL concentrations in collagen of these animals. Consistent with the similar growth rate in the two fatty groups, FL in skin collagen was the same for both groups, further demonstrating that PM had no effect on glycemic control. Fasting plasma insulin levels were 0.6 ± 0.1 µg/L in LN rats, measured at 0 weeks, and remained unchanged at 16 and 32 weeks. Plasma insulin levels were significantly higher in fa/fa rats at the beginning of the study (5.6 ± 0.3 µg/L) and rose to 7.1 ± 0.6 and 7.3 ± 0.6 µg/L in FC and FPM rats, respectively, at 32 weeks, without a significant change in blood glucose concentration. Thus, PM did not significantly affect insulin resistance in the fa/fa animals.

**Table 1. Glycemic control and glycation of protein in various groups of rats^{a}**

| | Week 0 | Week 16 | Week 32 |
|---|---|---|---|
| **Plasma glucose**^{**b**} | (mM) | | |
| **LN** | 6.7 ± 0.5 | 6.6 ± 0.5 | 6.6 ± 0.4 |
| **FC** | 7.2 ± 0.7 | 7.1 ± 0.4 | 6.9 ± 0.2 |
| **FPM** | 6.8 ± 0.5 | 6.9 ± 0.6 | 6.8 ± 0.5 |

| **Glycated Hemoglobin**^{**b**} | (Percent) | | |
|---|---|---|---|
| **LN** | 6.4 ± 0.5 | 6.3 ± 0.6 | 6.7 ± 0.3 |
| **FC** | 6.7 ± 0.2 | 6.6 ± 0.3 | 6.8 ± 0.2 |
| **FPM** | 6.8 ± 0.5 | 6.5 ± 0.3 | 6.8 ± 0.3 |

| **Glycation of Skin Collagen**^{**c**} | FL ( mmol/mol lysine) | | |
|---|---|---|---|
| | Week 0 | Week 11 | Week 32 |
| **LN** | 3.6 ± 0.2 | 4.0 ± 0.1 | 4.5 ± 0.3 |
| **FC** | 3.5 ± 0.2 | 3.0 ± 0.1* | 2.3 ± 0.2* |
| **FPM** | 3.5 ± 0.2 | 3.0 ± 0.1* | 2.3 ± 0.3* |

| | | | |
|---|---|---|---|
| ^{a} Data mean ± SD for 7 animals per group. | | | |
| ^{b}Plasma glucose and % total glycated hemoglobin are shown for 1, 4 and 8 months of the study, and are representative of data at all time points. Glucose and glycated hemoglobin were measured using commercial kits as described in Materials and Methods. | | | |
| ^{c} Skin biopsies were taken at week 0,11 and 32. Glycation (FL) was measured in insoluble collagen by SIM-GC/MS as described in Materials and Methods. * = p< 0.01 vs. LN rats and rats in same group at week 0. | | | |

Dyslipidemia. There were significant increases in plasma levels of triglycerides and total cholesterol of FC compared to the LN rats. Triglycerides in the LN group rose slightly during the first two months of the study, but remained at ~100 mg/dL·thereafter. In contrast, in FC rats they rose continuously throughout the study, reaching a mean value of ~800 mg/dL at 32 weeks. There was an early increase in triglycerides in the FPM group, however this rise was markedly blunted after 10 weeks. Thereafter, triglycerides remained in a relatively narrow range in the FPM group, about three times the level in LN animals, and less than 50% the values observed in FC animals. Plasma cholesterol in the LN group rose slightly through the course of the experiment, but was generally in the range of ~100 mg/dl. Cholesterol rose to approximately 250 mg/dL in FC rats, but was decreased to ~150 mg/dL in FPM rats, ~50% higher than in LN animals. As with triglycerides, there was a trend toward decreasing cholesterol in FPM rats after week 16. In summary, PM significantly reduced dyslipidemia, both triglycerides and cholesterol, in the fa/fa rat, without affecting overall weight gain, glycemia or insulin resistance.

Renal function. Total urinary protein and albumin and plasma creatinine were measured to evaluate the effect of PM on renal function in the obese animal model. Already by the sixth week of the study, both total urinary protein and albumin were rising in the FC compared to LN and FPM animals. Urinary albumin increased to 10-fold basal levels in FC vs. LN rats at 32 weeks, and was paralleled by comparable increases in total urinary protein. PM provided impressive protection against development of proteinuria in the fa/fa rat, an effect that was confirmed by measurement of PM on plasma creatinine concentration. Urinary volumes were 32.3 ml/day, 35.7 ml/day, and 27.0 ml/day, respectively, in LN, FC and FPM groups at the end of the study. Although urine volume was consistently greater in FC and FPM rats, compared to the LN group, there were no significant differences in urine volume between the FC and FPM animals at any point in the study.

Blood pressure and vascular changes. Animals in all groups had mean systolic blood pressure in the range of 125 -130 mm Hg at week 5 of the study. Systolic blood pressure rose gradually in the FC rats to 165 mm Hg, while it remained at initial values for FPM and LN groups, with no statistical differences between LN and FPM groups throughout the study. Vascular changes that occurred in the aorta and coronary arteries of the FC rats, compared to LN littermates, included proliferation of smooth muscle cells in the aorta and occlusion of the coronary arteries of the obese rats. These changes were observed in all FC animals. In contrast, there were minimal changes in the aortic or coronary vasculature of PM-treated fa/fa rats. The aorta of FPM rats were indistinguishable from those of LN animals in all but one animal. Despite apparent lipid vacuoles in the cytoplasm of FC rats, there was no evidence of atherosclerotic plaque or atherogenesis in the aorta of any of the Zucker rats.

Chemical modifications in skin collagen. The AGE/ALEs, CML and CEL, and the AGE pentosidine, increased modestly with age in LN animals, but at a significantly greater rate in the FC animals. Notably the increase in these compounds, including pentosidine which is derived exclusively from carbohydrates (7), occurred in the FC animals in the absence of hyperglycemia. PM treatment inhibited the increase in CML and CEL in skin collagen in fa/fa rats, and was also effective in limiting the increase in pentosidine through week 11 of the study, but by week 32 the significance of differences in pentosidine between FC and FPM rats was marginal (p = 0.07). MDA- and HNE-lysine represent chemical modifications that are uniquely derived from lipid peroxidation products, so that they can be clearly classified as ALEs. Neither ALE was detectable in skin collagen from LN animals at any time point. By 11 weeks 3 of 7 FC rats had measurable quantities of MDA-lysine in their skin collagen, and by 8 months this ALE was measurable in collagen of all FC animals (Table 2). In contrast no MDA-lysine was detectable in the FPM rats until 8 months, and then only in trace amounts (< 2 µmol/mol FL) in 3 of 7 animals. HNE-lysine was not measurable in LN animals in any skin sample; it was found in FC animals at 8 months, but was not detectable in FPM. Because of limited sample availability, collagen fluorescence was measured only in necropsy samples. As shown in Table 2, Maillard-type fluorescence was 2-fold higher in the FC compared to LN animals, and PM treatment prevented this increase.

**Table 2. Levels of ALEs and fluorescence in skin collagen of LN, FC and FPM rats**

| **Week #** | **Week 16** | **Week 32** | **Week 32** | **Week 32** |
|---|---|---|---|---|
| **Analyte** | MDA-Lys^{a} (mmol/mol Lys) | MDA-Lys (mmol/mol Lys) | HNE-Lys^{a} (mmol/mol Lys) | Fluorescence^{b} Arbitrary Units / mol FL |
| **LN** | ND^{c} | ND | ND | 0.05 ± 0.02 |
| **FC** | 0.57 ± 0.1 (3/7)^{d} | 0.68 ± 0.3 (7/7) | 0.011 ± 0.005 (7/7) | 0.10 ± 0.03^{e} |
| **FPM** | ND | Trace (3/7)^{d} | ND | 0.05 ± 0.02 |

| | | | | |
|---|---|---|---|---|
| ^{a} MDA- and HNE lysine were analyzed in skin collagen of all animals in each group by SIM-GC/MS. Numbers in parentheses indicate number of samples in which MDA- or HNE-lysine was measurable. | | | | |
| ^{b}Fluorescence was measured only in necropsy samples. | | | | |
| ^{c} ND = not detectable. | | | | |
| ^{d}MDA-lysine was detectable in only 3 of 7 FC animals at 16 weeks; mean of these three measurements is shown. Some FPM animals at week 32, but could not be reliably quantified. | | | | |
| ^{e} p < 0.05 vs. LN and FPM groups. | | | | |

## Claims

1. Use of pyridoxamine for the preparation of a pharmaceutical composition for treating or inhibiting obesity-related complications in an obese subject, wherein the obesity-related complications are selected from the group consisting of hyperlipidemia, renal disease, hypertension, and advanced lipoxidation end-product formation.

2. The use of claim 1 wherein the composition is to inhibit the obesity-related complications.

3. The use of claim 1 wherein the composition is to treat the obesity-related complications.

4. The use of any one of claims 1 to 3 wherein the obese subject is morbidly obese.

5. The use of any one of claims 1 to 3 wherein the obese subject is not hyperglycemic.

6. The use of claim 5 wherein the obese subject is insulin-resistant.

7. The use of any one of claims 1 to 3 wherein the obese subject suffers from a leptin receptor defect.

## Patentansprüche

1. Verwendung von Pyridoxamin zur Herstellung einer pharmazeutischen Zusammensetzung zur Behandlung oder Hemmung von mit Fettleibigkeit in Beziehung stehenden Komplikationen in einem fettleibigen Lebewesen, wobei die mit Fettleibigkeit in Beziehung stehenden Komplikationen ausgewählt sind aus der Gruppe bestehend aus Hyperlipidämie, Nierenerkrankung, Hypertension und fortgeschrittene Lipoxidation-Endproduktbildung (advanced lipoxidation end-product formation).

2. Verwendung nach Anspruch 1, wobei die Zusammensetzung die mit Fettleibigkeit in Beziehung stehenden Komplikationen hemmen soll.

3. Verwendung nach Anspruch 1, wobei die Zusammensetzung die mit Fettleibigkeit in Beziehung stehenden Komplikationen behandeln soll.

4. Verwendung nach einem der Ansprüche 1 bis 3, wobei das fettleibige Lebewesen krankhaft fettleibig ist.

5. Verwendung nach einem der Ansprüche 1 bis 3, wobei das fettleibige Lebewesen nicht hyperglykämisch ist.

6. Verwendung nach Anspruch 5, wobei das fettleibige Lebewesen gegenüber Insulin resistent ist.

7. Verwendung nach einem der Ansprüche 1 bis 3, wobei das fettleibige Lebewesen an einem Leptinrezeptor-Defekt leidet.

## Revendications

1. Utilisation de pyridoxamine pour la préparation d'une composition pharmaceutique pour le traitement ou l'inhibition de complications liées à l'obésité chez un sujet obèse, dans laquelle les complications liées à l'obésité sont choisies dans le groupe consistant en hyperlipidémie, affection rénale, hypertension, et formation de produit final de lipoxydation avancée.

2. Utilisation selon la revendication 1, dans laquelle la composition est destinée à inhiber les complications liées à l'obésité.

3. Utilisation selon la revendication 1, dans laquelle la composition est destinée à traiter les complications liées à l'obésité.

4. Utilisation selon l'une quelconque des revendications 1 à 3, dans laquelle le sujet obèse est obèse de manière morbide.

5. Utilisation selon l'une quelconque des revendications 1 à 3, dans laquelle le sujet obèse n'est pas hyperglycémique.

6. Utilisation selon la revendication 5, dans laquelle le sujet obèse est résistant à l'insuline.

7. Utilisation selon l'une quelconque des revendications 1 à 3, dans laquelle le sujet obèse souffre d'un défaut de récepteur de leptine.
